(19) Europäisches Patentamt
European Patent Office
Office européen des brevets

(11) EP 0 672 729 B1

(12) EUROPÄISCHE PATENTSCHRIFT

(45) Veröffentlichungstag und Bekanntmachung des Hinweises auf die Patenterteilung:
**15.09.1999 Patentblatt 1999/37**

(51) Int Cl.[6]: **C09B 57/00**, C09B 67/10, C07D 487/04, C08K 5/3415 // (C07D487/04, 209:00, 209:00)

(21) Anmeldenummer: **95810149.5**

(22) Anmeldetag: **08.03.1995**

(54) **Diketopyrrolopyrrolpigment mit hoher Farbtonreinheit**

Diketopyrrolopyrrol pigment with a high hue purity

Pigment de dicetopyrrolopyrrol avec une grande pureté de teinte

(84) Benannte Vertragsstaaten:
**BE CH DE FR GB IT LI NL**

(30) Priorität: **16.03.1994 CH 78094**

(43) Veröffentlichungstag der Anmeldung:
**20.09.1995 Patentblatt 1995/38**

(73) Patentinhaber: **Ciba Specialty Chemicals Holding Inc.**
**4057 Basel (CH)**

(72) Erfinder:
- **Wooden, Gary, Dr.**
  **CH-1716 Oberschrot (CH)**
- **Schlöder, Ingo**
  **CH-1753 Matran (CH)**
- **Wallquist, Olof, Dr.**
  **CH-1723 Marly (CH)**

(56) Entgegenhaltungen:
**EP-A- 0 181 290** **EP-A- 0 184 982**
**EP-A- 0 190 999** **EP-A- 0 302 018**
**EP-A- 0 337 435**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 672 729 B1

## Beschreibung

Die vorliegende Erfindung betrifft eine neue Pigmentform von 1,4-Diketo-3,6-bis-(biphenyl-4-yl)-pyrrolo[3,4-c]pyrrol mit unerwartet hoher Farbtonreinheit.

1,4-Diketo-3,6-bis(biphenyl-4-yl)-pyrrolo[3,4-c]pyrrol ist aus US-Patent 4 579 949 bekannt. Es handelt sich dabei um eine eher transparente Form mit einer für bestimmte Einsätze unzureichende Farbtonreinheit. In US-Patent 4 931 566 wird eine neue Methode zur Herstellung von chemisch reineren Pyrrolo[3,4-c]pyrrolen mit ebenfalls verbesserten koloristischen Eigenschaften, u.a. auch verbesserte Sättigung, d.h. Farbtonreinheit, beschrieben, die darin besteht, die Protolyse des Pigmentalkalisalzes sequentiell, d.h. in mindestens 2 Portionen, durchzuführen. Das in diesem Patent beschriebene 1,4-Diketo-3,6-bis(biphenyl-4-yl)-pyrrolo[3,4-c]pyrrol zeichnet sich durch eine deutlich verbesserte Deckkraft und eine etwas bessere, aber immer noch nicht ganz befriedigende Farbtonreinheit aus. Für bestimmte Anwendungen, wie z.B. in Kunststoffen und vor allem in nichtmetallisierten Automobillacken und Industrielacken, ist eine möglichst hohe Sättigung von wesentlicher Bedeutung. In jüngerer Zeit ist demnach die Nachfrage nach hochwertigen Pigmenten mit besonders hoher Sättigung erheblich gestiegen. Einige Diketopyrrolopyrrolpigmente haben sich als hochwertige Pigmente bewährt, es stellte sich aber das Problem, auch von bereits so hochgepriesenen Pigmenten eine Form mit besonders hoher Sättigung zu erhalten.

Es ist nun gefunden worden, dass bei der Herstellung von 1,4-Diketo-3,6-bis(biphenyl-4-yl)-pyrrolo[3,4-c]pyrrol überraschenderweise ein Produkt mit hoher Deckkraft und ganz unerwartet hoher Sättigung entsteht, wenn die Protolyse des als Zwischenprodukt entstehenden Pigmentsalzes und die anschliessende Konditionierung durch Austragen der Pigmentsalzsuspension in einem Niederalkylalkohol bei einer Temperatur zwischen 65 und 150°C in einer Portion durchgeführt wird.

Die vorliegende Erfindung betrifft demnach eine Pigmentform des Diketopyrrolopyrrols der Formel

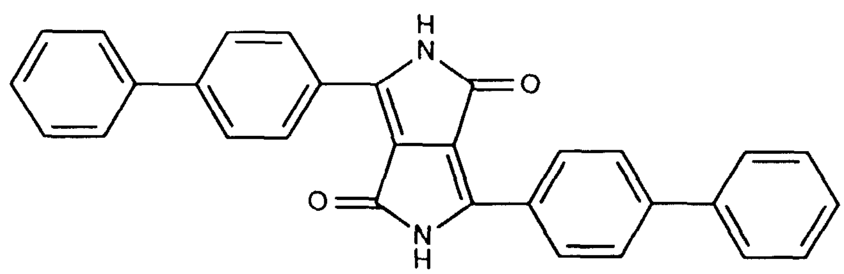

(I),

mit hoher Sättigung, gekennzeichnet durch ein Chroma $C^*_{ab}$ von mindestens 45 im CIELAB-System in einer gemäss DIN 53775, Teil 2 hergestellten Vollton-PVC-P-Pressfolie mit einer Pigmentkonzentration von 1 % und 1,0 mm Dicke.

Bevorzugte Diketopyrrolopyrrole der Formel I werden gekennzeichnet durch

- ein Chroma $C^*_{ab}$ von mindestens 46, insbesondere zwischen 46,3 und 54,
- eine Helligkeit L* von mindestens 36, insbesondere zwischen 36,5 und 42 und
- einen Farbwinkel $h_{ab}$ von mindestens 23, insbesondere zwischen 23,4 und 29

im CIELAB-System, in einer wie oben beschrieben hergestellten PVC-P-Folie.

Die im CIELAB-System verwendeten Begriffe Chroma oder Buntheit ($C^*_{ab}$), Helligkeit (L*) und Farb- oder Bunttonwinkel ($h_{ab}$) sind aus der Literatur bekannt, beispielsweise aus H.G. Völz, Industrielle Farbprüfung, Grundlagen und Methoden, VCH Verlagsgesellschaft mbH, Weinheim, D, 1990. Es sei hier nur hervorgehoben, dass die Begriffe Chroma bzw. Buntheit der Sättigung entsprechen.

Das erfindungsgemässe Diketopyrrolopyrrol zeichnet sich selbstverständlich nicht nur in PVC, sondern in jedem Substrat zu dessen Pigmentierung es geeignet ist, durch die unerwartet hohe Sättigung aus. PVC ist im vorliegenden Fall lediglich als Richtsubstrat für die quantitative Bestimmung der Sättigung gewählt worden, weil entsprechende Probekörper (Pressfolien) nach einer einfachen Norm (DIN 53775, Teil 2) hergestellt werden können. Wie bereits angedeutet, zeigt das erfindungsgemässe Pigment auch in anderen Kunststoffen sowie in Lacken eine überraschend hohe Sättigung.

Die Herstellung des erfindungsgemässen Produktes, die neu und demnach ebenfalls Gegenstand vorliegender Erfindung ist, erfolgt durch Umsetzung von 1 Mol eines Bernsteinsäure-dicyclohexylesters, -dialkylesters, -monoalkylmonophenylesters oder -diphenylesters, wobei im Bernsteinsäureesterrest Alkyl $C_1$-$C_{18}$-Alkyl, und Phenyl unsub-

stituiertes Phenyl oder durch ein oder zwei Halogenatome, eine oder zwei $C_1$-$C_6$-Alkyl- oder $C_1$-$C_6$-Alkoxygruppen substituiertes Phenyl bedeuten, mit 2 Mol eines Nitrils der Formel

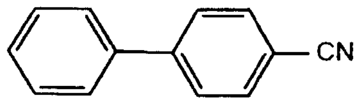

(II)

in einem inerten organischen Lösungsmittel in Gegenwart eines Alkalimetalls oder eines Alkali-alkoholates als starke Base bei erhöhter Temperatur zu einem Pigmentalkalimetallsalz und anschliessende Freisetzung einer Verbindung der Formel I durch Protolyse des entstandenen Pigmentalkalimetallsalzes und nachfolgende Konditionierung, dadurch gekennzeichnet, dass die Pigmentalkalisalz-Suspension einem Gemisch aus Wasser und einem Alkohol ROH, worin R $C_2$-$C_4$-Alkyl ist, bei einer Temperatur zwischen 65 und 150°C zugegeben und 30 Minuten bis 24 Stunden ebenfalls bei einer Temperatur zwischen 65 und 150°C behandelt wird.

$C_1$-$C_6$-Alkyl bedeutet z.B. Methyl, Ethyl, n-Propyl, Isopropyl, n-Butyl, sec.-Butyl, tert.-Butyl, n-Amyl, tert.-Amyl, Hexyl, und $C_1$-$C_{18}$-Alkyl zusätzlich z.B. Heptyl, Octyl, 2-Ethylhexyl, Nonyl, Decyl, Dodecyl, Tetradecyl, Hexadecyl oder Octadecyl;

$C_1$-$C_6$-Alkoxy bedeutet beispielsweise Methoxy, Ethoxy, n-Propoxy, Isopropoxy, Butyloxy oder Hexyloxy.

Beim Alkohol ROH handelt es sich z.B. um Isopropanol oder n-Butanol, aber ganz besonders bevorzugt um n-Propanol und insbesondere Ethanol.

Bevorzugt wird die Pigmentalkalisalz-Suspension in ein Wasser/Alkohol-Gemisch ausgetragen, wobei es sich zweckmässig um ein Gemisch von Wasser und Alkohl im Verhältnis 5-50:95-50, bevorzugt 10-30:90-70 Vol% handelt.

Die Protolyse und die anschliessende Konditionierung erfolgen in basischem bis neutralem Milieu. Bevorzugt wird ein basisches Milieu und ein Temperaturbereich zwischen 70 und 115°C, während 1 bis 8 Stunden.

Bei den zu verwendenden Bernsteinsäuredialkyl- oder -diphenylestern kann es sich um symmetrische oder asymmetrische Diester handeln. Bevorzugt verwendet man aber symmetrische Bernsteinsäurediester, insbesondere symmetrische Bernsteinsäuredialkylester. Liegt ein Bernsteinsäurediphenyl- oder -monophenylmonoalkylester vor, so kann Phenyl beispielsweise unsubstituiertes oder durch ein oder zwei Halogenatome, wie Chlor, $C_1$-$C_6$-Alkylgruppen, wie Methyl, Ethyl, Isopropyl oder tert.-Butyl, oder $C_1$-$C_6$-Alkoxygruppen, wie Methoxy oder Ethoxy, substituiertes Phenyl bedeuten. Phenyl bedeutet bevorzugt unsubstituiertes Phenyl. Handelt es sich um einen Bernsteinsäuredialkyl- oder -monoalkylmonophenylester, so kann Alkyl unverzweigt oder verzweigt sein, bevorzugt verzweigt, und vorzugsweise 1 bis 12, insbesondere 1 bis 8 und besonders bevorzugt 1 bis 5 C-Atome enthalten. Verzweigtes Alkyl ist bevorzugt sek.- oder tert.-Alkyl, wie z.B. Isopropyl, sek.-Butyl, tert.-Butyl und tert.-Amyl. Ganz bevorzugt verwendet man symmetrische verzweigte Bernsteinsäuredialkylester, worin jeder Alkylrest im Bernsteinsäureesterrest 3 bis 5 C-Atome aufweist.

Beispiele für Bernsteinsäurediester sind Bernsteinsäuredimethylester, -diethylester, -dipropylester, -dibutylester, -dipentylester, -dihexylester, -diheptylester, -dioctylester, -diisopropylester, -di-sec.-butylester, -di-tert.-butylester, -di-tert.-amylester, -di-[1,1-dimethylbutyl]-ester, -di-[1,1,3,3-tetramethylbutyl]-ester, -di-[-1,1-dimethylpentyl]-ester, -di-[1-methyl-1-ethyl-butyl]-ester, -di-[1,1-diethylpropyl]-ester, -diphenylester, -di-[4-methylphenyl]-ester, -di-[2-methylphenyl]-ester, -di-[4-chlorphenyl]-ester, -di-[2,4-dichlorphenyl]-ester und -monoethyl-monophenylester.

Die oben aufgeführten Bernsteinsäurediester und das Nitril der Formel II sind bekannte Verbindungen und können nach bekannten Verfahren hergestellt werden.

Man führt die Umsetzung des Bernsteinsäurediesters mit dem Nitril der Formel II in einem organischen Lösungsmittel durch. Als Lösungsmittel eignen sich beispielsweise primäre, sekundäre oder tertiäre Alkohole mit 1 bis 10 C-Atomen, wie Methanol, Ethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Ethyl-3-pentanol und 2,4,4-Trimethyl-2-pentanol, Glykole, wie Ethylenglykol oder Diethylenglykol, ferner Ether, wie Tetrahydrofuran oder Dioxan, oder Glykolether, wie Ethylenglykol-mono- oder -di-methylether, Ethylenglykol-mono- oder -di-ethylether, Diethylenglykol-monomethylether oder Diethylenglykolmonoethylether, ferner dipolar-aprotische Lösungsmittel, wie Dimethylformamid, N,N-Dimethylacetamid, Nitrobenzol und N-Methylpyrrolidon, aliphatische oder aromatische Kohlenwasserstoffe, wie Benzol oder durch Alkyl, Alkoxy oder Halogen substituiertes Benzol, wie Toluol, Xylole, Anisol oder Chlorbenzol, oder aromatische N-Heterocyclen, wie Pyridin, Picolin oder Chinolin. Die oben genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Im erfindungsgemässen Verfahren verwendet man als Lösungsmittel bevorzugt einen Alkohol, insbesondere einen sekundären oder tertiären Alkohol. Bevorzugte tertiäre Alkohole sind tert.-Butanol und tert-Amylalkohol. Dabei sind auch Gemische davon, oder Gemische dieser bevorzugten Lösungsmittel mit aromatischen Kohlenwasserstoffen,

wie Toluol oder Xylole, oder mit durch Halogen substituierten Benzolen, wie Chlorbenzol oder o-Dichlorbenzol, von grossem Interesse.

Anmeldungsgemäss geeignete starke Basen sind Alkalimetalle, wie Lithium, Natrium und Kalium, und Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, -n-butylat, -sek.-butylat, -tert.- butylat, -2-methyl-2-butylat, -2-methyl-2-pentylat, -3-methyl-3-pentylat und -3-ethyl-3-pentylat. Man kann aber auch ein Gemisch der oben genannten Alkalialkoholate verwenden. Bevorzugt verwendet man Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kaliumisopropylat, -sek.-butylat, -tert.-butylat und -tert-amylat. Dabei können die Alkalialkoholate auch in situ hergestellt werden, indem man den entsprechenden Alkohol mit dem Alkalimetall umsetzt.

Im erfindungsgemässen Verfahren kann man die starke Base beispielsweise in einer Menge von 0,1 bis 10 Mol, bevorzugt 1,9 bis 4,0 Mol, bezogen auf 1 Mol des Bernsteinsäurediesters, einsetzen. Obwohl grundsätzlich stöchiometrische Mengen an Base genügen, beeinflusst in vielen Fällen ein Basenüberschuss die Ausbeute günstig.

Die Umsetzung kann beispielsweise bei einer Temperatur von 60 bis 140°C, vorzugsweise aber von 80 bis 120°C, durchgeführt werden.

Zur Umsetzung des Bernsteinsäurediesters mit dem Nitril der Formel II ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zuzugeben. Eine bevorzugte Ausführungsform, die sich in der Regel besonders günstig auf die Ausbeute auswirkt, besteht darin, dass man das umzusetzende Nitril zusammen mit der starken Base vorlegt und den Bernsteinsäurediester im Bereiche der Reaktionstemperatur zudosiert. Eine weitere Möglichkeit besteht darin, dass man den Bernsteinsäurediester und das umzusetzende Nitril gleichzeitig zur vorgelegten Base zudosiert. Es ist durchaus möglich, das erfindungsgemässe Verfahren nicht nur batchweise, sondern auch kontinuierlich durchzuführen.

Insbesondere bei Bernsteinsäurediestern mit niederen Alkylresten und bei Alkoholaten, die sich aus niederen Alkoholen ableiten, wie z.B. Methanol, Ethanol, n-Propanol, Isopropanol oder tert.-Butanol, kann es sich als vorteilhaft erweisen, den bei der Umsetzung entstehenden niederen Alkohol laufend aus dem Reaktionsmedium zu entfernen, um höhere Ausbeuten zu erzielen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Zur Protolyse des erhaltenen Pigmentsalzes wird das Pigmentalkalisalz zu dem aus Wasser und Alkohol bestehenden Protolysemittel zugegeben. Nach der Behandlung der erhaltenen Suspension bei einer Temperatur zwischen 65 und 150°C während 30 Minuten bis 24 Stunden fällt das erfindungsgemässe Pigment der Formel I aus und kann durch an sich bekannte Trennverfahren, wie Abfiltrieren, isoliert werden. Das Wasser/Alkohol-Gemisch kann in beliebigen Mischungsverhältnissen von 5 bis 20 Gew.Teilen auf 1 Teil des entstandenen Pigmentalkalisalzes eingesetzt werden.

Das erfindungsgemässe Pigment eignet sich ausgezeichnet zum Pigmentieren von hochmolekularem organischen Material. Es lässt sich beispielsweise in Form von Pulver, Teig, Flushpaste und Zubereitung anwenden und eignet sich z.B. für Druckfarben, Leimfarben, Binderfarben oder Lacke aller Art, wie physikalisch und oxydativ trocknende Lacke, säure-, amin- und peroxidhärtende Lacke oder Polyurethanlacke. Das Pigment kann auch zum Färben von synthetischen, halbsynthetischen oder natürlichen makromolekularen Stoffen verwendet werden, wie Polyvinylchlorid, Polystyrol, Polyolefine, wie z.B. Polyethylen und Polypropylen und ferner Polyestern, Phenoplasten, Aminoplasten und Gummi. Weitere Anwendungsgebiete sind das Färben von natürlichen, regenerierten oder künstlichen Fasern, wie Glas-, Silikat-, Asbest-, Holz-, Cellulose-, Acetylcellulose-, Polyacrylnitril-, Polyester-, Polyurethan- und Polyvinylchloridfasern oder deren Gemischen, eventuell zusammen mit anderen organischen oder anorganischen Pigmenten. Man erhält mit dem erfindungsgemässen Pigment Drucke, Lackierungen, Anstriche, Beläge, Beschichtungen, geformte Gebilde, wie Folien, Fäden, Platten, Blöcke, Granulate und Stäbe, mit brillanter roter Farbe von hervorragender Dauerhaftigkeit.

Das erfindungsgemässe rote Pigment kann zum Färben von festen, elastischen, pastenartigen, dickflüssigen, dünnflüssigen oder thixotropen Massen verwendet und in diese nach an sich bekannten Verfahren eingearbeitet werden. Wasserhaltige Teige können beispielsweise durch Einrühren des Pigments in Wasser, gegebenenfalls unter Zusatz eines Netz- oder Dispergiermittels oder durch Einrühren oder Einkneten des Pigments in ein Dispergiermittel in Gegenwart von Wasser und gegebenenfalls von organischen Lösungsmitteln oder Oelen erhalten werden. Diese Teige können beispielsweise wiederum zur Herstellung von Flushpasten, Druckfarben, Leimfarben, Kunststoffdispersionen und Spinnlösungen verwendet werden. Das Pigment kann aber auch durch Einrühren, Einwalzen, Einkneten oder Einmahlen in Wasser, organische Lösungsmittel, nicht trocknende Oele, trocknende Oele, Lacke, Kunststoffe oder Gummi gebracht werden. Schliesslich ist es auch möglich, das Pigment durch trockenes Mischen mit organischen

oder anorganischen Massen, Granulaten, Faserstoffen, Pulvern und anderen Pigmenten zu Stoffmischungen zu verarbeiten.

Das erfindungsgemässe Pigment zeichnet sich nicht nur durch die Reinheit des Farbtons mit sehr hoher Sättigung, bei guter Deckkraft und hervorragender Farbstärke, sondern auch durch gute Allgemeinechtheiten, wie Licht- und Wetterbeständigkeit, Ueberlackierechtheit, Migrations- und Hitzebeständigkeit, sowie durch gute rheologische Eigenschaften aus.

Das erfindungsgemässe Pigment ist vorzugsweise für die Färbung von Polyolefinen und insbesondere von wässrigen und/oder Lösungsmittel enthaltenden Lacken, insbesondere Automobillacken, geeignet.

Die nachfolgenden Beispiele erläutern die Erfindung.

Beispiel 1a): 9,2 g Natrium werden zu 160 ml trockenem tert.-Amylalkohol gegeben, auf 100°C geheizt und kräftig unter Rückfluss bis zur vollständigen Lösung gerührt. Die Lösung wird auf 90°C gekühlt, dann werden 35,85 g 4-Biphenylnitril zugegeben, wieder auf Rückflusstemperatur (ca. 110°C) geheizt und langsam (ca. 6 $^1/_2$ Stunden) 24,4 g Bernsteinsäurediisopropylester zugegeben. Danach wird die Suspension weitere zwei Stunden am Rückfluss erhitzt, dann auf 100°C gekühlt und mit 20 ml tert.-Amylalkohol verdünnt.

b) Die so erhaltene Pigmentsalz-Suspension wird einem Gemisch aus 70 ml Wasser und 490 ml n-Propanol zugegeben, dann 6 Stunden bei 85°C gerührt, auf 40°C abgekühlt und filtriert. Der Rückstand wird zuerst mit Methanol bis das Filtrat farblos ist und dann mit Wasser, ebenfalls bis das Filtrat farblos ist, gewaschen und im Vakuumtrockenschrank bei 80°C getrocknet. Man erhält 27,5 g eines roten Produktes der Formel

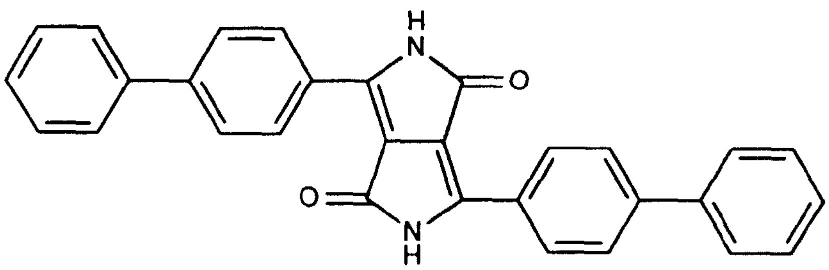

in Pulverform.

Mit dem erhaltenen Produkt wird nach der in DIN 53775, Teil 2 festgelegten Methode eine PVC-P-Pressfolie von 1,0 mm Dicke (vgl. Punkt 6.3 von DIN 53775, Teil 2) mit 1 % Pigmentkonzentration hergestellt und die Farbwerte nach CIELAB bestimmt.

Die erhaltenen Werte sind wie folgt:

$L^* = 38{,}4$  $C^*_{ab} = 48{,}1$  $h_{ab} = 25{,}0$

Beispiel 2a): 26,7 g Natrium werden zu 460 ml trockenem tert.-Amylalkohol gegeben, auf 100°C geheizt und kräftig unter Rückfluss bis zur vollständigen Lösung gerührt. Die Lösung wird auf 90°C gekühlt, dann werden 105,3 g 4-Biphenylnitril zugegeben, wieder auf Rückflusstemperatur (ca. 110°C) geheizt und langsam 70,4 g Bernsteinsäurediisopropylester zugegeben, wobei ein Gemisch aus tert.-Amylalkohol und dem sich bildenden Isopropanol (334 g) fortwährend abdestilliert und gleichzeitig mit 335 g tert.-Amylalkohol ersetzt wird.

b) Die erhaltene Pigmentsalz-Suspension wird auf 85°C abgekühlt und einem Gemisch aus 1450 ml Ethanol und 290 ml Wasser zugegeben, dann wird 6 Stunden unter Rückfluss gerührt und anschliessend auf 40°C abgekühlt und filtriert. Der Rückstand wird zuerst mit Methanol, bis das Filtrat farblos ist, und dann mit Wasser, ebenfalls bis das Filtrat farblos ist, gewaschen und im Vakuumtrockenschrank bei 80°C getrockent. Man erhält 107 g des gleichen roten Produktes, wie in Beispiel 1.

Mit dem erhaltenen Produkt wird nach der in DIN 53775, Teil 2 festgelegten Methode eine PVC-P-Pressfolie von 1,0 mm Dicke (vgl. Punkt 6.3 von DIN 53775, Teil 2) mit 1 % Pigmentkonzentration hergestellt und die Farbwerte nach CIELAB bestimmt.

Die erhaltenen Werte sind wie folgt:

$L^* = 39{,}2$  $C^*_{ab} = 50{,}3$  $h_{ab} = 25{,}1$

Beispiel 3: Die Pigmentsalz-Suspension wird wie in Beispiel 1a beschrieben hergestellt und einem Gemisch aus 500 ml Isopropanol und 100 ml Wasser zugegeben. Die erhaltene Suspension wird in einem Autoklaven 5 Stunden bei 120°C erhitzt, dann auf Zimmertemperatur abgekühlt, filtriert und wie in Beispiel 1 beschrieben gewaschen und getrocknet. Man erhält 30,0 g des roten Pigments in Pulverform.

Mit dem erhaltenen Produkt wird nach der in DIN 53775, Teil 2 festgelegten Methode eine PVC-P-Pressfolie von 1,0 mm Dicke (vgl. Punkt 6.3 von DIN 53775, Teil 2) mit 1 % Pigmentkonzentration hergestellt und die Farbwerte nach CIELAB bestimmt.

Die erhaltenen Werte sind wie folgt:
$L^* = 36{,}9$ $C^*_{ab} = 46{,}4$ $h_{ab} = 23{,}5$

Beispiele 4 und 5: Eine Mischung von

| | | |
|---|---|---|
| 230 g | Glasperlen (Ø = 2 mm) | |
| 92 g | eines thermohärtenden Acryllackes bestehend aus | |
| | 57,80 g | Acrylharz ®URACRON 2263 XB, 50 %ig in Xylol/Butanol (Chem. Fabrik Schweizerhalle), |
| | 10,35 g | Melaminharz ®CYMEL 327 90 %ig in Isobutanol (Dyno Cyanamid), |
| | 5,50 g | Butylglycolacetat |
| | 11,40 g | Xylol |
| | 3,30 g | n-Butanol |
| | 1,00 g | Siliconöl, 1 %ig in Xylol |
| | 2,65 g | Dispergiermittel ®Disperbyk 160 (Byk Chemie) und |
| 8 g | Pigment | |

werden 90 Minuten in einer Dispergiermaschine dispergiert. Nach Abtrennen der Glasperlen wird der Farblack auf Aluminiumbleche deckend gespritzt. Der Lack wird 30 Minuten bei Raumtemperatur abdunsten gelassen und anschliessend 30 Minuten bei 115°C eingebrannt.

Durch Farbmessung gemäss CIELAB-System werden die $C^*_{ab}$-, $L^*$- und $h_{ab}$-Werte der erhaltenen Lackierungen bestimmt. Sie sind in nachstehender Tabelle aufgeführt.

| Beispiel | Pigment | $C^*_{ab}$ | $L^*$ | $h_{ab}$ |
|---|---|---|---|---|
| 4 | aus Beispiel 1 | 45,3 | 37,0 | 23,4 |
| 5 | aus Beispiel 2 | 47,5 | 38,6 | 24,7 |

Alle Farbmessungen wurden mittels eines Minolta CM-2002® Spectrophotometers (d/8 Geometrie, unter Einschluss des Glanzes, Lichtart D65, Beobachter 10°) ausgeführt.

**Patentansprüche**

1. Pigmentform des Diketopyrrolopyrrols der Formel

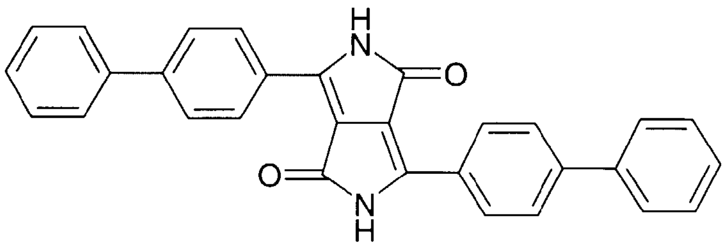

(I),

mit hoher Sättigung, gekennzeichnet durch ein Chroma $C^*_{ab}$ von mindestens 45 im CIELAB-System in einer gemäss DIN 53775, Teil 2 hergestellten Vollton-PVC-Pressfolie mit 1% Pigmentkonzentration und 1,0 mm Dicke.

2. Pigmentform gemäss Anspruch 1, gekennzeichnet durch

ein Chroma $C^*_{ab}$ von mindestens 46,
eine Helligkeit $L^*$ von mindestens 36 und
einen Farbwinkel $h_{ab}$ von mindestens 23.

3. Pigmentform gemäss Anspruch 1, gekennzeichnet durch

ein Chroma $C^*_{ab}$ zwischen 46,3 und 54
eine Helligkeit L* zwischen 36,5 und 42 und
einen Farbwinkel $h_{ab}$ zwischen 23,4 und 29.

**4.** Verfahren zur Herstellung der hochdeckenden Pigmentform des Diketopyrrolopyrrols, gemäss Anspruch 1, durch Umsetzung von 1 Mol eines Bernsteinsäuredicyclohexylesters, -dialkylesters, -monoalkylmonophenylesters oder -diphenylesters. wobei im Bernsteinsäureesterrest Alkyl $C_1$-$C_{18}$-Alkyl, und Phenyl unsubstituiertes oder durch ein oder zwei Halogenatome, eine oder zwei $C_1$-$C_6$-Alkyl- oder $C_1$-$C_6$-Alkoxygruppen substituiertes Phenyl bedeuten, mit zwei Mol eines Nitrils der Formel

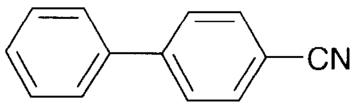

(II)

in einem inertem organischen Lösungsmittel in Gegenwart eines Alkalimetalls oder eines Alkali-alkoholates als Base bei erhöhter Temperatur zu einem Pigmentalkalimetallsalz und anschliessende Freisetzung einer Verbindung der Formel I durch Protolyse des entstandenen Pigmentalkalimetallsalzes und nachfolgende Konditionierung, dadurch gekennzeichnet, dass die Pigmentalkalisalz-Suspension einem Gemisch aus Wasser und einem Alkohol ROH, worin R $C_2$-$C_4$-Alkyl ist, bei einer Temperatur zwischen 65 und 150 °C zugegeben und 30 Minuten bis 24 Stunden ebenfalls bei einer Temperatur zwischen 65 und 150 °C behandelt wird.

**5.** Verfahren gemäss Anspruch 4, dadurch gekennzeichnet, dass es sich beim Alkohol ROH um Ethanol oder n-Propanol handelt.

**6.** Verfahren gemäss Anspruch 4, dadurch gekennzeichnet, dass die Pigmentalkalisalz-Suspension in ein Wasser/ Alkohol-Gemisch, bei einem Verhältnis Wasser zu Alkohol von 5-50:95-50 Vol% ausgetragen wird.

**7.** Verfahren gemäss Anspruch 4, dadurch gekennzeichnet, dass Protolyse und Konditionierung im basischen Milieu bei einer Temperatur zwischen 70 und 115 °C während 1 bis 8 Stunden durchgeführt werden.

**8.** Hochmolekulares organisches Material enthaltend ein Diketopyrrolopyrrolpigments gemäss Anspruch 1.

**9.** Hochmolekulares organisches Material gemäss Anspruch 8, dadurch gekennzeichnet, dass es sich um ein Polyolefin handelt.

**10.** Hochmolekulares organisches Material gemäss Anspruch 8, dadurch gekennzeichnet, dass es sich um einen Lack handelt.

**11.** Hochmolekulares organisches Material gemäss Anspruch 10, dadurch gekennzeichnet, dass es sich um einen Automobillack handelt.

## Claims

**1.** A pigment form of the diketopyrrolopyrrole of formula

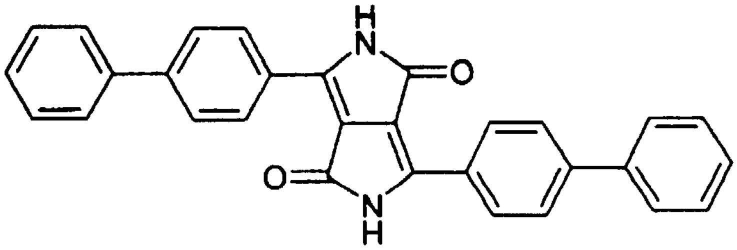

(I),

having high saturation, characterized by a chroma $C^*_{ab}$ of at least 45 in the CIELAB system in a full-shade PVC pressed sheet prepared in accordance with DIN 53 775, Part 2, and having a pigment concentration of 1% and a thickness of 1.0 mm.

**2.** A pigment form according to claim 1, characterized by

- a chroma $C^*_{ab}$ of at least 46,
- a lightness L* of at least 36, and
- a hue angle $h_{ab}$ of at least 23,

**3.** A pigment form according to claim 1, characterized by

a chroma $C^*_{ab}$ from 46.3 to 54,
a lightness L* from 36.5 to 42, and
a hue angle $h_{ab}$ from 23.4 to 29.

**4.** A process for the preparation of the highly opaque pigment form of the diketopyrrolopyrrole according to claim 1, by reacting 1 mol of dicyclohexyl succinate, dialkyl succinate, monoalkyl monophenyl or diphenyl succinate, in which in the succinic ester radical alkyl is $C_1$-$C_{18}$alkyl and phenyl is unsubstituted phenyl or phenyl which is substituted by one or two halogen atoms, one or two $C_1$-$C_6$alkyl or $C_1$-$C_6$alkoxy groups, with 2 mol of a nitrile of formula

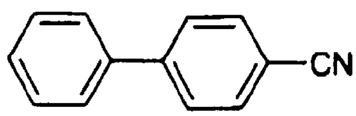

(II)

in an inert organic solvent and in the presence of an alkali metal or of an alkali metal alcoholate as base, at elevated temperature, to give a pigment alkali metal salt, and subsequently liberating a compound of formula I by protolysis of the resultant pigment alkali metal salt and subsequent conditioning, which comprises adding the suspension of said pigment alkali metal salt to a mixture of water and an alcohol ROH, wherein R is $C_2$-$C_4$alkyl, at a temperature from 65 to 150°C and treating the resulting mixture for 30 minutes to 24 hours likewise at a temperature from 65 to 150°C.

**5.** A process according to claim 4, wherein the alcohol ROH is ethanol or n-propanol.

**6.** A process according to claim 4, wherein the pigment alkali metal salt suspension is discharged into a water/alcohol mixture in a ratio of water to alcohol of 5-50:95-50% by volume.

**7.** A process according to claim 4, wherein protolysis and conditioning are carried out in a basic medium at a temperature from 70 to 115°C for 1 to 8 hours.

**8.** A high molecular weight organic material comprising a diketopyrrolopyrrole pigment according to claim 1.

**9.** A high molecular weight organic material according to claim 8, which is a polyolefin.

**10.** A high molecular weight organic material according to claim 8, which is a coating material.

**11.** A high molecular weight organic material according to claim 10, which is an automotive coating material.

**Revendications**

**1.** Forme pigmentaire du dicétopyrrolopyrrole de formule

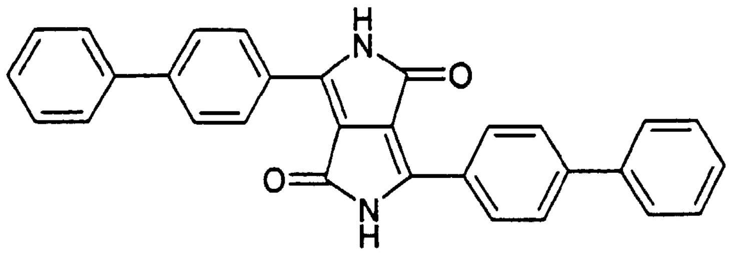

(I)

ayant une saturation élevée, caractérisée par une chromie $C^*_{ab}$ d'au moins 45 mesurée selon le système CIELAB dans une feuille pressée de PVC à teinte homogène, ayant une teneur de 1 % en pigment et une épaisseur de 1,0 mm, préparée selon la norme DIN 53775, partie 2.

**2.** Forme pigmentaire selon la revendication 1, caractérisée par

une chromie $C^*_{ab}$ d'au moins 46,
une luminance L* d'au moins 36 et
un angle de chromovirage $h_{ab}$ d'au moins 23.

**3.** Forme pigmentaire selon la revendication 1, caractérisée par

une chromie $C^*_{ab}$ comprise entre 46,3 et 54
une luminance L* comprise entre 36,5 et 42 et
un angle de chromovirage $h_{ab}$ comprise entre 23,4 et 29.

**4.** Procédé de préparation d'une forme pigmentaire à grand pouvoir couvrant du dicétopyrrolopyrrole selon la revendication 1, par transformation de 1 mole d'un ester dicyclohexylique, d'un ester dialkylique, d'un ester monoalkylmonophénylique ou d'un ester diphénylique de l'acide succinique, alkyle représentant dans le reste ester de l'acide succinique alkyle en $C_1$-$C_{18}$ et phényle représentant phényle non substitué ou phényle substitué par un ou deux atomes d'halogène, un ou deux groupes alkyle en $C_1$-$C_6$ ou alkoxy en $C_1$-$C_6$, avec 2 moles d'un nitrile de formule

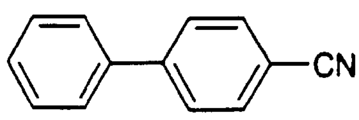

(II)

dans un solvant inerte, en présence d'un métal alcalin ou d'un alcoolate de métal alcalin comme base forte, à température élevée, pour donner un sel pigmentaire de métal alcalin, et par libération ultérieure d'un composé de formule (I) par protolyse du sel pigmentaire de métal alcalin et par conditionnement ultérieur, caractérisé en ce que l'on ajoute la suspension de sel pigmentaire de métal alcalin à un mélange d'eau et d'un alcool ROH, dans lequel R représente un groupe alkyle en $C_2$-$C_4$, à une température comprise entre 65 et 150°C et on traite pendant une durée de 30 minutes à 24 heures également à une température comprise entre 65 et 150°C.

**5.** Procédé selon la revendication 4, caractérisé en ce qu'il s'agit pour l'alcool ROH de l'éthanol ou du n-propanol.

**6.** Procédé selon la revendication 4, caractérisé en ce que l'on extrait la suspension du sel pigmentaire alcalin dans

un mélange eau/alcool, pour un rapport de l'eau à l'alcool de 5-50:95-50 % en vol.

7. Procédé selon la revendication 4, caractérisé en ce que l'on met en oeuvre la protolyse et le conditionnement en milieu basique à une température comprise entre 70 et 115°C pendant 1 à 8 heures.

8. Matière organique de haut poids moléculaire contenant un pigment de dicétopyrrolopyrrole selon la revendication 1.

9. Matière organique de haut poids moléculaire selon la revendication 8, caractérisée en ce qu'il s'agit d'une polyoléfine.

10. Matière organique de haut poids moléculaire selon la revendication 8, caractérisée en ce qu'il s'agit d'un vernis.

11. Matière organique de haut poids moléculaire selon la revendication 8, caractérisée en ce qu'il s'agit d'un vernis pour automobile.